# EUROPÄISCHE PATENTSCHRIFT

(11) **EP 3 866 688 B1**
(45) Veröffentlichungstag und Bekanntmachung des Hinweises auf die Patenterteilung: **09.08.2023**
(21) Anmeldenummer: 19863974.2
(22) Anmeldetag: 03.12.2019
(51) Int. Cl.: A61B 5/103, A61B 5/00, A61F 5/01

(54) **BELASTUNGSERFASSENDE ORTHESE**
LOAD-DETECTING ORTHESIS
ORTHÈSE À DÉTECTION DE CHARGE

(30) Priorität: 03.12.2018 DE 102018220853
(43) Veröffentlichungstag der Anmeldung: 25.08.2021
(73) Patentinhaber: Golex AG, 4052 Basel (CH)
(72) Erfinder: WERNER, Frank, 3182 Ueberstorf (CH)
(74) Vertreter: Pietruk, Claus Peter
(86) Internationale Anmeldenummer: PCT/IB2019/001419
(87) Internationale Veröffentlichungsnummer: WO 2020/115561

(56) Entgegenhaltungen:
- EP-A1- 2 783 630
- WO-A2-01/39655
- DE-A1-102013 001 193
- US-A1- 2008 167 580
- US-A1- 2014 195 023

## Beschreibung

Die vorliegende Erfindung betrifft das oberbegrifflich Beanspruchte und bezieht sich somit auf die Erfassung einer Belastung.

Nach Knochenbrüchen und Operationen im Bein- bzw. Fußbereich treten oftmals Komplikationen auf. So verheilen Brüche nicht, sondern müssen neuerlich operiert werden, Endoprothesen wachsen nicht ein, sondern lockern sich etc. Die medizinische Praxis zeigt, dass nach bestimmten orthopädischen Eingriffen bei etwa 10% aller Patienten neue Operationen nur deshalb erforderlich werden, weil der Patient die kritische Stelle überlastet hat.

Es gibt daher bereits eine Vielzahl von Vorschlägen, wie eine Überlastung erkannt und vor ihr gewarnt werden kann.

Vom vorliegenden Erfinder ist etwa die CH 704 972 bekannt, wonach der Bewegungsapparat im postoperativen Verlauf bei Sprunggelenk, Knie und Hüfte für mehrere Wochen nur teilbelastet werden darf. Es wurde daher vorgeschlagen, dass der Patient eine spezielle Orthese trägt, in der sich eine Messanordnung befindet, mit der Kraft-Zeit-Kurven für jeden Schritt erfasst werden können. Es wird vorgeschlagen, dass Belastungsgrenzen für einen Trainingsmodus und einen maximalen Belastungswert über Schalter einstellbar sind und dann, wenn eine Belastung im richtigen Bereich erfolgt, ein positives Feedback in Form einer grün leuchtenden LED und beim Überschreiten eines eingestellten Wertes eine Warnung durch eine rote LED in Kombination mit einem Tonsignal oder Vibration erfolgt.

Aus der DE 37 14 218 A I ist eine therapeutische Schutzvorrichtung gegen Überlastung des menschlichen Bewegungsapparates bekannt, die als sog. "Fußsohlenwaage" bezeichnet wird, wobei die Verwendung nur weniger Drucksensoren wie zwei Drucksensoren im Ballenbereich und eines Drucksensors im Fersenbereich in einer Einlegesohle vorgeschlagen wird. Es wird ausgeführt, dass die Auswertung eines Verhaltensprofils die Heilungschancen für den Patienten verbessert, und dass eine Mindestbelastung den Heilungsvorgang durch mechanische Reize, zu stimulieren vermag. Es wird weiter darauf hingewiesen, dass zu Anfang der Behandlung ein niedriger Soll-Belastungsbereich vorgegeben werden kann. Eine Belastungsgeschichte von z.B. 2 Wochen soll aufzuzeichnen sein.

Aus der DE 198 10 182 C1 ist eine Vorrichtung zum Erfassen einer Überlastung des unteren Bewegungsapparates und/oder der Wirbelsäule einer Person bekannt, wobei auf Endoprothesen Bezug genommen wird und darauf, dass eine möglichst frühe gerichtete und dosierte Belastung dem Heilungsprozess zuträglich sei, aber Überlastungen durch harte Stöße beim schnellen Treppen laufen oder Springen zu vermeiden seien. Es wird davon gesprochen, dass eine Überlastung von der lasterfassenden Anordnung registriert wird, wenn ein vorbestimmter Lastschwellwert überschritten wird bzw. der Lastschwellwert auch so gewählt werden kann, dass das Signal bereits dann ausgelöst wird, wenn die Belastung zwar noch nicht schädigend ist, eine weitere Erhöhung der Belastung jedoch schädigend sein würde. Weiter wird erwähnt, dass Lastschwellwerte individuell einstellbar sind, beispielsweise angepasst an die jeweiligen osteologischen, muskulären, neurologischen Verhältnisse und/oder die Bänderverhältnisse der Person.

Aus der DE 295 12 711 U1 ist ein Messsystem zur statischen und dynamischen Druckverteilungsmessung an der Fußsohle des Menschen bekannt. Es wird eine Datenerfassungseinheit vorgeschlagen, mit der Drucksensoren an den Kraftmesssohlen abgefragt und Messdaten über Funk übertragen werden können. Die Messergebnisse sollen in Form von farbigen Isobaren oder als Druckgebirge dargestellt werden, wodurch "einerseits die Übertragung per Funk möglich" sei und "die Belastungen an der Fußsohle mit ausreichend hoher Auflösung gemessen werden können". Vorgeschlagen werden beispielsweise 64 Drucksensoren, die eine Feinauflösung von 1/16 N/cm² ermöglichen sollen und mit mindestens 40 Hz für langsame und normale Gehbewegungen und 50 bis 100 Hz für schnelle Bewegungen wie sie bei sportmedizinischen Untersuchungen auftreten, abgetastet werden sollen.

Ein Drucksensor, z.B. für ein Fußbekleidungsstück, ist aus der DE 11 2013 002 836 entspreehend WO 2013/182 633 bekannt. Darin sollen Drucksensoren bereitgestellt werden, deren Druckmesszellen einen vergrößerten dynamischen Bereich aufweisen, wobei deren elektrischer Widerstand mit zunehmendem Druck langsamer, aber über einen weiteren Bereich abnimmt.

Verwiesen wird auch auf die DE 20 2017 000 608 U1, die bereits eine belastungserfassende Orthese zeigt, mit welcher vor Spitzenbelastungen gewarnt wird.

Die WO 2015/145 273 A1 offenbart ein System zur Beurteilung der Belastung einer unteren Extremität mit Beschleunigungsmesser, um Schockwellen aus einzelnen Fuß-Auftritten zu erfassen. Es sollen Schock wellen-Daten erzeugt und verarbeitet werden, um den Benutzer bei der künftigen Belastungsminimierung zu unterstützen.

Die DE 299 19 839 U1 befasst sich mit einem System zur Erfassung von Trittkräften, bei welchem ein Patient die Belastung innerhalb ärztlich empfohlener Grenzen halten soll. Es wird zwar das Speichern eines zeitlichen Verlaufs der erfasst Werte der Trittkräfte erwähnt und die Gewinnung von Kraft-Zeit-Diagrammen, eine besonders empfehlenswerte Signalauswertung, die noch dazu batterieschonend ist, ist dem Dokument jedoch nicht entnehmbar. Weiter betrifft die WO 01/39655A3 eine Schuhsensoranordnung zur Ganganalyse.

Aus der DE 10 2013 001193A1 ist ein Informationssystem und ein Verfahren zur Information eines Patienten über seine Auftrittsbelastung bekannt, dass eine Orthese, eine Drucksensoreinrichtung, einer auswertende Einrichtung, eine Energieversorgungseinrichtung und eine Informationsübermittlungseinrichtung aufweist. Mit dieser Anordnung soll der Patient bei Überschreiten eines Sollwertes der Auftrittsbelastung informiert werden.

Aus der EP 2783630A1 ist ein Verfahren und eine Vorrichtung zur Analyse menschlicher Bewegung bekannt. Es sollen Messdaten von einem Drucksensor in einer Einlegesohle oder einem gesammelt werden und aus der Bearbeitung der Messdaten sollen Bewegung bzw. Haltung eines Menschen hergeleitet werden.

Ein Rehabilitations- System ist aus der US 2008/0167580A1 bekannt. Dabei soll ebenfalls die Belastung in einem Schuh ermittelt werden.

Aus der US 2014/0195023A1 ist ein Verfahren und eine ein System bekannt, dass einem Läufer eine Rückmeldung über seinen Laufstil geben soll.

Es ist wünschenswert, eine belastungserfassende Orthese anzugeben, die bei preiswerter Ausbildung gleichwohl präzise Warnungen ausgeben kann. Überdies ist es wünschenswert, Fehlalarme zu vermeiden. Weiter ist es alternativ und oder zusätzlich wünschenswert, einen langfristigen Betrieb einer belastungserfassenden Orthese ohne Batteriewechsel bzw. Nachladen von Akkumulatoren ermöglichen.

Die Aufgabe der Erfindung besteht darin, Neues für die gewerbliche Anwendung bereitzustellen.

Die Lösung dieser Aufgabe wird in unabhängiger Form beansprucht. Einige bevorzugte Ausführungsformen sind in den Unteransprüchen angegeben.

Gemäß einem ersten wichtigen Aspekt wird somit eine belastungserfassende Orthese mit einem Sensorsignale erzeugenden Sensor, der Sensorelemente in Druckzonen aufweist, und einer Sensorsignale auswertenden Elektronik dafür vorgeschlagen, die dazu ausgebildet ist, auf kritische Belastungen hinzuweisen, wobei die Sensorsignale auswertende Elektronik dazu ausgebildet ist, Kennwerte zu mittels der Sensorelemente zumindest druckzonenweise erfassten Druckbelastungsereignissen zu ermitteln, einen Summenwert zu ermitteln, in welchen die Kennwerte nach Größe derart gewichtet eingehen, dass wenigstens drei unterschiedliche Gewichte verwendet werden, und ein Hinweissignal zu erzeugen, wenn der Summenwert eine festgelegte Größe überschreitet.

Eine derart gestaltete Orthese hat den Vorteil, dass ein Benützer einerseits zuverlässig gewarnt werden kann, wenn die Art und Weise seiner Orthesenbenützung gesundheitlich abträglich ist, andererseits zugleich jedoch vermieden wird, dass ein Benützer durch weitgehend singuläre Ereignisse und nachdem diese aufgetreten sind, zusätzlich verunsichert wird. Häufig treten übermäßige Druckbelastungsereignisse nämlich dann auf, wenn ein Benützer beispielsweise stolpert und sich abfangen muss, unerwartet auszuweichen hat usw. Gerade bei Personen, die nicht an Orthesen gewohnt sind, kann dies kritisch sein. In derartigen Situationen ist die Erzeugung eines Aufmerksamkeit erfordernden Warnsignals absolut kontraproduktiv, weil es in derartigen Situationen nicht darauf ankommt, den Benützer vor der ihm als kritisch bekannten Situation zu warnen, sondern ihm die Gelegenheit zu geben, die kritische Situation möglichst gut und somit ablenkungsfrei zu bereinigen.

Indem nicht ein Einzelereignis ausgewertet wird, sondern Summenwerte aus den Ereignissen, wird dies deutlich erleichtert, was insbesondere dann gilt, wenn, wie vorgesehen, die Kennwerte von Druckbelastungsereignissen gewichtet werden. Dies wird im Regelfall dazu führen, dass eine kritische hohe Belastung, die dauerhaft beobachtet wird, zur Warnsignalerzeugung führt, wohingegen singuläre, auch hohe Ereignisse zwar registriert werden, jedoch eben, nicht unmittelbar zu Warnungen führen müssen. Indem mehr als drei unterschiedliche Gewichtungen verwendet werden, ist trotz geringen Aufwandes eine bereits hinreichend feine Einteilung des Einflusses einer kurzzeitigen Überlastung oder kurzzeitigen hohen Belastung möglich. In einer bevorzugten Ausführungsform ist bei der belastungserfassenden Orthese bevorzugt, wenn die Sensorsignale auswertende Elektronik weiter dazu ausgebildet ist, die Kennwerte durch Vergleich mit einer Mehrzahl Schwellwerte zu klassifizieren, insbesondere je Schritt, und die Klassifikation derart zur Gewichtung heranzuziehen, dass zu größeren Kennwerten hin stärkere Gewichtungen erhalten werden.

Es wird also nicht einfach eine "zu hoch / unkritisch"-Bewertung vorgenommen, sondern es wird genauer analysiert, wie kritisch sich eine momentane Druckbelastung tatsächlich auswirkt. Das Hinweissignal wird daher typisch ein Warnsignal sein; es könnte aber auch ein Anreizsignal für wiederholt korrekte Belastung darstellen. Die Größe, die der Summenwert überschreiten muss, damit das Hinweissignal erzeugt wird, ist typisch einstellbar, könnte jedoch auch vorab fest und unveränderlich implementiert sein.

Typische Gehmuster führen zu einer Druckbelastung im Fußbereich, bei der die unterschiedlichen Bereiche des Fußes immer wieder in bestimmter Abfolge vergleichsweise hoch belastet werden. Diese Belastungsmuster lassen sich sehr gut verwenden, um Schritte zu erkennen, zum Teil sogar dergestalt, dass erkannt werden kann, ob eine Person horizontal, aufwärts oder abwärts läuft und/oder Treppen auf- oder absteigt. Daneben ist auch eine Identifizierung der Schwungphase eines Beines während der Bewegung möglich und es können Zeiten erkannt werden, in denen eine Person z.B. ruhig steht oder sitzt. Es ist möglich und sinnvoll, den Zeitverlauf von Sensorsignalen mit einer derart hohen zeitlichen Auflösung zu erfassen, dass derartige Ereignisse voneinander unterschieden werden können bzw. zumindest einige der genannten Ereignisse identifizierbar sind. Es sei darauf hingewiesen, dass eine Auswertung von Sensorsignaldruckkurven beinweise möglich ist, also für den linken und rechten Fuß jeweils völlig unabhängig voneinander durchgeführt werden kann, dass aber gleichfalls und sogar bevorzugt auch eine gemeinsame Auswertung erfolgen kann, weil beim Gehen typisch ein Bein während der Schwungphase nicht belastet wird, während der gegenüberliegende Fuß zu diesen Zeiten höher belastet wird, und zwar beim ansonsten gesunden Benützer dergestalt, dass eine Abrollbewegung über die Fußsohle hinweg erfolgt, weshalb es vorteilhaft ist, wenn Sensorelemente in mehreren Druckzonen vorgesehen sind.

Die Schritterkennung hilft insbesondere, wiederholt zu hohe Belastungen bei Standardsituationen wie Gehbewegungen zu unterscheiden von zu hohen Spitzenbelastungen durch singuläre Ereignisse. Dies kann bei der Gewichtung ebenfalls herangezogen werden, weil sich womöglich auch eine die sinnvolle Sollbelastung in geringem Maß überschreitende Belastung beim Schreiten stärker auswirkt als einzelne hohe Singulärbelastungen.

Es sei des weiteren betont, dass in der typischen und vorteilhaften Implementierung nicht nur die Grenzen bzw. Schwellen der Summenwerte einstellbar sind, ab welchen ein Hinweis-Signal erzeugt wird, sondern dass auch die Gewichtung vorteilhaft einstellbar ist. So kann dafür gesorgt werden, dass Belastungen an bestimmten Fußzonen als besonders kritisch bewertet werden, was für bestimmte Behandlungen besonders vorteilhaft ist, etwa nach Mittelfußbrüchen. Hier kann durch entsprechende Auswertung bzw. Gewichtung der Druckzonen eine genauere Anpassung an die jeweiligen Erfordernisse des Patienten erfolgen.

Es sei im Übrigen darauf hingewiesen, dass die Hinweissignale auch dann in leicht verständlicher Form ausgegeben werden können, wenn eine Vorwarnung ausgegeben werden muss, etwa weil eine bestimmte Belastung zwar kritisch, aber noch nicht hochgefährlich ist. In einem solchen Fall kann etwa so lange ein grünes Licht angezeigt werden, wie der Summenwert eine 1. Schwelle noch nicht überschritten hat; das grüne Licht kann etwa als grüne LED, die erregt wird, implementiert sein oder etwa als grüne Fläche auf einem Display wie der Anzeigefläche einer Smartwatch oder eines Smartphones. Dann, wenn der Summenwert eine L Schwelle überschreitet, kann stattdessen ein gelbes Licht angezeigt werden, wiederum beispielsweise durch Erregen einer gelben LED oder durch Anzeigen einer gelben Fläche auf einem Display. Sobald der Summenwert auch eine 2. Schwelle überschreitet. die größer als die 1. Schwelle ist, wird dann bevorzugt stattdessen ein rotes Licht angezeigt, wiederum beispielsweise durch eine LED oder eine Leuchtfläche auf einem Display. Dass die LEDs oder Flächen räumlich wie bei einer Ampel angeordnet werden können, sei erwähnt. Dem Benützer wird somit die Möglichkeit gegeben, jederzeit sein Laufverhalten durch einen kurzen Blick zu überprüfen und insbesondere selbst dann, wenn keine Warnungen ausgegeben werden müssen, das einwandfreie Funktionieren zu erkennen.

Es sei darauf hingewiesen, dass die Hinweissignal-Erzeugung nicht nur dann ausgelöst werden kann, wenn der Summenwert eine für die Gesundheit des Benutzers kritische Größe in nachteiliger Weise überschreitet, sondern dass gegebenenfalls eine Hinweissignal-Erzeugung auch dann erfolgen kann, wenn wiederholt und in der erfindungsgemäß gewichteten Weise eine korrekte Belastung festgestellt wurde. Dies ist zunächst bei sehr vielen Patienten sinnvoll, weil viele Heilungsprozess tatsächlich durch eine regelmäßige, allerdings vergleichsweise geringe Belastung begünstigt werden. Es könnte bei einer Ampelanordnung, wie sie vorstehend beschrieben wurde, demgemäß etwa auch ein gelbes Licht angezeigt werden, solange ein Benutzer sich kaum oder nur in unzureichendem Maße bewegt; wenn er eine ausreichende Bewegung aufnimmt und etwa eine in einem bestimmten Zeitraum vorgegebene Schrittzahl erreicht hat, ohne sich zu überlasten, könnte die Anzeige auf Grün wechseln; wo eine Vorwarnung erforderlich ist, wäre eine gelb-rote Anzeige, beispielsweise durch simultanes Erregen zweier Display-Felder oder zweier LEDs möglich, und sobald eine hochkritische Belastungssituation mit häufiger zu hoher Überlastung erfasst wird, kann eine ausschließlich rote Anzeige erfolgen.

Eine das korrekte Verhalten eines Patienten bestärkende Hinweissignalerzeugung kann beispielsweise auch dort erfolgen, wo Patienten eine auf Dauer für sie günstigere Gangart antrainiert werden soll; erwähnt sei etwa, dass Patienten mit Cerebral-Parese eine als "Steppergang" bezeichnete Abrollbewegung des Fußes ausführen, was auf Dauer zu erheblichen Problernen fuhrt. Diesbezüglich sei erwähnt, dass Kinder, die über einen längeren Zeitraum auf dem Vorderfuß laufen, als "gewohnheitsmäßige Zehenspitzengänger" bezeichnet werden. Besteht diese Angewohnheit über einen längeren Zeitraum, kann es zu strukturellen Veränderungen am wachsenden Skelett kommen; zudem wirkt das Gangbild mit zunehmendem Alter stigmatisierend. Der Zehenspitzengang bleibt zwar nur bei etwa 20 Prozent der Betroffenen über das 10. Lebensjahr hinaus bestehen; gleichwohl ist es sinnvoll, betroffene Kinder frühzeitig zu therapieren, da früher einsetzende Behandlungen zu größeren Behandlungserfolgen führen. Die Behandlung ist allerdings schwierig, da Kinder die Gangstörung zumindest kurzzeitig willentlich unterdrücken und ein Gangbild mit Fersenerstkontakt zeigen können, was die Objektivierung der Pathologie erschwert und klare Kriterien zur Bewertung oder Einteilung der Gangstörung verhindert. Dies spiegelt sich zugleich in Therapiekonzepten, bei denen es an einer klaren Empfehlung mangelt und die daher derzeit unter anderem auf Dehnübungen, Gangschulung, Einlagen oder Orthesen setzen, ebenso wie auf eine operative Wadenmuskulatur-Verlängerung oder die Injektion von Botulinum-Toxin A.

Mit der erfindungsgemäßen Orthese kann derartigen Patienten ein für sie günstigeres, normales Laufverhalten antrainiert werden, wozu die erfindungsgemäße, belastungserfassende Orthese, mit der druckzonenweise eine Belastung ermittelt und ein Summenwert für eine Vielzahl nacheinander auftretender Druckbelastungsereignisse, gewichtet bestimmt wird, einsetzbar ist. Dabei kann etwa ein positives Rückkopplungssignal an einen jungen, eine gesündere, normale Gangart trainierenden Patienten als Hinweissignal ausgegeben werden. Es sei darauf hingewiesen, dass die Größe, die der Summenwert überschreiten muss, um eine Hinweissignalerzeugung auszulösen, je nach Trainingsfortschritt variieren kann. So kann zu Beginn eines Trainings ein Hinweissignal bereits nach wenigen, womöglich unmittelbar aufeinanderfolgenden Schritten mit korrekter Belastung, wie 3-5 Schritten, erzeugt werden, wohingegen ein Hinweis Signal nach längerem Training erst erzeugt wird, wenn eine größere Zahl von Schritten wie 10, 20,50 oder 100 Schritte mit korrekter Belastung bzw. mit nur wenigen zwischenzeitlich falschbelastenden Schritten während der Schritt-Folge absolviert wird. Eine entsprechende Anpassung, wann ein den Patienten bestärkendes, positives Hinweissignal ausgegeben wird, kann durch Veränderung der festgelegten Größe vorgenommen werden, beispielsweise von einer Physiotherapeutin. Die Anzahl der Schritte, die fehlerfrei absolviert werden muss, bevor ein Hinweis Signal erzeugt wird, kann dabei noch im Hinblick darauf bestimmt sein, wie präzise die Einzelschritte einem korrekten Schritt entsprechen, d. h. wie genau die am Fuß ermittelte Druckbelastung jener eines korrekten Geh-Verhaltens entspricht. Dies ist durch die Gewichtung ohne weiteres möglich. Es kann gegebenenfalls auch das Auswerte- Schema verändert werden, etwa dahingehend, dass ein bereits akkumuliert Wert immer dann zurück auf Null gesetzt wird, wenn entweder ein einzelner Fehltritt mit falschem Abrollverhalten bzw. falschem Druckbelastungsmuster erfasst wurde - in diesem Fall beginnt die notwendige Anzahl nacheinander fehlerfrei zu absolvierende Schritte von neuem - oder ein den Patienten bestärkendes, positives Hinweissignal ausgegeben wurde.

Zugleich kann erfasst werden, ob die Belastungen in den einzelnen Druckzonen nur näherungsweise oder sehr gut einem korrekten Gang entsprechen; dies kann durch die Gewichtung im Summenwert berücksichtigt werden.

Für eine spätere Trainingsphase kann ein Zähler für falsche und ein Zähler für korrekte Belastungen implementiert sein, die beide mit Schwellwerten verglichen werden. In derartigen Fällen kann gegebenenfalls, gerade bei Patienten mit weit fortgeschrittenem Training, auf eine mechanische Unterstützung weitgehend oder vollständig verzichtet werden; die Orthese-Wirkung, eine korrekte Fußstellung beim Laufen zu gewährleisten, ergibt sich demgemäß aus der akustischen, optischen oder taktilen Rückkopplung an den Benutzer vermittels des Hinweissignals. In typischen Fällen wird die Orthese jedoch eine mechanisch unterstützende Orthese sein, die dazu ausgebildet ist, eine Extremität, insbesondere einen Fuß und/oder Knöchel und/oder Unterschenkel mechanisch so zu unterstützen, dass eine anatomisch korrekte Position gehalten oder eingenommen wird. Erwähnt sei, was derartige Zähler angeht, auch, dass die Erfassung ordnungsgemäßer Schritte einerseits und überlastender Schritte andererseits vorteilhaft sein kann, um zu ermitteln, ob genügend positive Stimuli für einen günstigen Heilungsverlauf erzeugt werden. Dass gegebenenfalls etwa bei besonders schweren, etwa adipösen Patienten andere Schwellwerte verwendbar sind, sei erwähnt.

In einer besonders bevorzugten Variante wird mit ausgewertet, ob kurz hintereinander sehr hohe Werte auftreten oder immer wieder, aber in einzelnen kurzen, zeitlich weit auseinander liegenden Episoden. So kann es sein, dass eine Person Einkäufe nach Hause trägt und sich dabei deutlich zu stark belastet, was zu einer hohen zeitlichen Dichte sehr großer Kennwerte führt, oder dass angelegentlich einzelne Stufen genommen werden, was ebenfalls zu höherer Belastung führt, insbesondere bei Personen, die noch nicht erfahren genug im Umgang mit Gehhilfen (Krücken) sind, wobei die Überlastungsereignisse bei Treppensteigen weniger oft nacheinander auftreten als etwa jene durch das Tragen von Einkäufen.

Die Berücksichtigung der zeitlichen Dichte von Kennwerten oberhalb bestimmter Schwellwerte kann etwa durch Abklingenlassen von Druckbelastungs-Beiträgen zum Summenwert geschehen, durch Berechnung eines gleitenden Mittelwertes bzw. durch schnelleres oder alleiniges Abklingenlassen solcher Druckbelastungsereignisse, die zwar nicht mehr rein heilungsförderlich sind, jedoch maximal für sich allein als noch nicht als kritisch bewertet werden müssen- es könnten also z.B. die absolut unkritischen Beiträge und die einzeln noch unkritischen Beiträge abklingen, während hochkritische nicht abklingen. So kann beispielsweise ein Summenwert berechnet werden, bei dem die einzelnen Beiträge zu Gesamtsumme nicht nur entsprechend der Größe der Kennwerte gewichtet werden, sondern bei jedem Kennwert zusätzlich noch das Alter berücksichtigt wird. Was das Abklingen angeht, so kann der Beitrag eines Kennwertes zum Summenwert beispielsweise linear mit der Zeit abnehmen, insbesondere auf 0 für sehr alte Kennwerte, oder auf einen festen, geringen Wert, sodass etwa Überlastungen während der unmittelbar vorangegangenen Schritte, beispielsweise der unmittelbar vorangegangenen 5. 10 oder 20 Schritte z.B. doppelt so hoch gewichtet werden wie die 5,10 oder 20 Schritte vor den unmittelbar vorangegangenen; die 5,10 oder 20 Schritte wiederum davor können wiederum halb so hoch gewichtet werden usw., was sich fortsetzt, bis eine Gewichtung auf nur noch ein Prozent, 5 Prozent, 10 Prozent oder dergleichen bezogen auf die Bedeutung der unmittelbar vorher erfolgten Schritte abgefallen ist. Die Bedeutung sehr weit zurück liegender Überlastungen für den Summenwert klingt demgemäß ab. Erwähnt sei, dass geringfügige Überlastungen schneller abklingen können als höhere Überlastungen bzw. das besonders hohe Überlastungen überhaupt nicht abzuklingen brauchen. Es sei erwähnt, dass andere Abklingverhalten als die explizit erwähnten sich ebenfalls implementieren lassen. Es wird aus der obigen Offenbarung ersichtlich sein, dass das Abklingenlassen ein durch digitale Datenverarbeitung, insbesondere durch geeignete, auf Kennwerte einwirkende Multiplikationsoperationen, implementierter Prozess ist und insoweit insbesondere explizit durchzuführen und zu implementieren ist. Zu beachten ist im Übrigen, dass das Abklingen zusätzlich zur Gewichtung der Kennwerte erfolgt. Dies ist vor allen Dingen dort von Bedeutung, wo das Abklingenverhalten durch die Gewichtung beeinflusst ist, weil Druckereignisse unterschiedlicher Gewichtung unterschiedlich schnell abklingen, sollen.

In einer besonders bevorzugten Variante werden für die Gewichtung wenigstens 4, bevorzugt wenigstens 5 Schwellwerte verwendet. Neben dem Schwellwert "unkritisch" und dem Schwellwert "extrem kritisch", der für Druckbelastungen gewählt werden sollte, die binnen kürzester Zeit, also innerhalb weniger Schritte, zu einem ganz erheblichen Anstieg einer Heilungsgefährdung führen, können Schwellwerte z.B. für eine nur geringe Überschreitung der Sollbelastung und für eine erhebliche, für sich alleine aber noch nicht unmittelbar direkte Konsequenzen durch einzelne Episoden bewirkende Druckbelastungen unterschieden werden. Wenn etwa eine heilungsfordernde Belastung bei 10 kg und eine bekannt übermäßige Belastung bei 30 kg liegt, können Zwischenschwellen bei etwa 17 kg und 25 kg gewählt werden; bei genauer gewünschter Auflösung können die Schwellwerte in jeweils 5 kg-Schritten oder feiner festgelegt werden. Die Gewichtung erfolgt bevorzugt für die geringfügigen Belastungsüberschreitungen mindestens linear, bevorzugt mehr als linear, also etwa quadratisch, so dass für eine einfache Überschreitung ein Wert von 1 zur Summe hinzuaddiert wird, für eine etwas höhere Überschreitung ein Wert von 2 und für eine besonders große Überschreitung ein Wert von 3 oder 5 zum Summenwert addiert wird. Es wird aber darauf hingewiesen, dass ein oder der sehr große Wert nicht zwingend einen sehr viel größeren Summen Beitrag liefern muss.

Die belastungserfassende Orthese kann ohne weiteres preiswert ausgebildet werden, weil eine spezifische Anpassung an unterschiedliche Patienten im Regelfall entbehrlich ist. Es reicht dadurch, dass nur wenige Druckzonen in flächenintegrativer bzw. über bestimmte Flächenmittelnder Weise ausgewertet werden, bereits ein Standardsensor für praktisch alle Patienten, zumindest alle Patienten einer Größengruppe. So wird es möglich, etwa Einlegesohlen zu entwerfen, die lediglich an eine Schuhgröße und die Verwendung für den linken bzw. rechten Schuh angepasst sein müssen, wobei sogar eine Standardisierung auf eine von mehreren Schuhgrößengruppen ohne Weiteres möglich ist.

In einer entsprechenden Einlegesohle oder dergleichen bzw. einer dieser verwendenden Orthese brauchen nicht mehr als vier, bevorzugt nicht mehr als lediglich drei, insbesondere lediglich zwei Druckzonen vorgesehen sein. Im Regelfall wird es ausreichend sein, wenn eine Druckzone unterhalb der Ferse angeordnet ist und eine zweite Druckzone beim Ballen. Am Ballen können zwei einzelne Sensoren bzw. kleine Sensorfelder vorgesehen sein, um an unterschiedlichen Stellen des Ballens, also einerseits näher am Außenrist und andererseits näher am Inhezirist, Messungen vornehmen zu können. Dort, wo mehr als zwei Druckzonen vorgesehen sind, können insbesondere am Außenrist mittig zwischen Ferse und Ballen sowie nahe der Fußwölbung, insbesondere bei Personen mit mangelhafter Wölbung, Druckzonen vorgesehen sein, was gegebenenfalls gerade bei Patienten mit Vorbelastungen hilfreich sein kann, um ein kritisches Gangmuster bzw. die für die Benutzung bestimmter Prothesen oder Endoprothesen besonders kritischen Belastungsmuster zu erkennen.

Es wird bevorzugt sein, je Druckzone nicht mehr als vier, insbesondere nicht mehr als drei, insbesondere ein oder zwei Sensorelemente vorzusehen. Die Reduktion der Sensorelementzahl verringert nicht nur den baulichen Aufwand, sondern trägt auch dazu bei, dass die auszuwertende Datenmenge gering bleibt; durch die vorgeschlagene Gewichtung der Kennwerte und die Summenwertbildung ist diese Reduktion ausgewerteter Informationen unkritisch für die Erkennung kritischer Belastungen. Die angesichts der geringen Sensorelementzahl erforderliche elektronische Auswertung insbesondere durch Analog/Digital-Wandlung ist vorteilhaft besonders wenig aufwendig und lässt sich somit mit nicht nur preiswerten, sondern auch energiesparend zu betreibenden integrierten Schaltkreisen leicht bewerkstelligen. Die Summenwertbildung und Gewichtung ist, auch dann, wenn ein Abklingen berücksichtigt wird, gleitende Mittelwerte gebildet werden usw., regelmäßig energetisch sehr unkritisch, weil es selbst dort, wo Schrittmuster erkannt werden müssen und dafür etliche Digital werte je Schritt, beispielsweise zwischen 16 und 512 Werte je Halbschritt auszuwerten sind, ausreicht, die aus einem Schritt heraus bestimmte Kennwertgröße zu ermitteln, was durch Maximalbetrachtung. Flächenintegration nahe beim oder um das Maximum usw. möglich ist, und danach nur noch sehr wenige Werte betrachtet werden müssen, die zudem langsam, nämlich allenfalls mit etwa der Geschwindigkeit der Schrittabfolge verarbeitet werden müssen. Die erforderliche Taktrate einer Datenverarbeitungsanordnung ist somit besonders gering, was bekannterweise den Energieverbrauch senkt.

Es ist nicht nur möglich, sondern sogar günstig, wenn auch gegebenenfalls nicht zwingend, den Drucksensor bzw. die einzelnen Sensorelemente in einer Belastungen vergleichmässigenden bzw. Druckspitzen dämpfenden Einlegesohle anzuordnen. Dies hat den Vorteil, dass bezüglich der Datenauswertung der Aufwand für eine Mittelwertbildung dadurch verringert werden kann, dass weniger Werte zu betrachten sind, und dass weiter die zu betrachtende Dynamik bei der Analog/Digital-Wandlung geringer sein braucht und Unterschiede von Benützer zu Benützer geringer ausfallen. Eine Vergleichmäßigung durch die Einlegesohlen ist sowohl räumlich als auch, weil belastungsdämpfend, zeitlich druckverteilend.

Obwohl es möglich ist, die Sensoren in Standardeinlegesohlen einzubauen, können auch für jeweilige Patienten dedizierte Einlegesohlen, etwa zum Ausgleich von Plattfüßen, Knick- und Senkfußstellungen usw., verwendet werden. Da die Einlegesohle die Druckspitzen vergleichsmäßigt und Aufstandskräfte räumlich zumindest teilweise verteilt, ist die genaue Lage der Sensoren vergleichsweise unkritisch und kann insbesondere ohne Weiteres mit geringstem Aufwand, falls erforderlich, kalibriert werden, so dass eine Verwendung in Orthopädiefachbetrieben leicht möglich ist. Es ist also typisch eine Druckbelastungen vergleichmäßigende, elastische Trittdämpfung in der Sohle vorhanden.

Es ist bevorzugt, wenn die Auswerteelektronik zwei Bereiche besitzt, nämlich einerseits sensornahe Schaltkreise, in denen die Drucksignale aus den Sensorelementen konditioniert, digitalisiert und gegebenenfalls partiell ausgewertet werden, etwa durch (gewichtete) Summenbildung, und dann über eine insbesondere drahtlose Schnittstelle an einen separaten Empfänger gespeist werden. Die Aufteilung dergestalt, dass eine erhebliche Vorauswertung lokal fußnah erfolgt, hat den Vorteil, dass die energieaufwändige Datenübertragung nur selten und für wenig Daten benötigt wird. Es ist insbesondere nicht erforderlich, regelmäßig Daten zu übertragen, solange diese unkritisch sind. Vielmehr ist eine Zwischenspeicherung fußsohlennah ohne Weiteres möglich, da die ausgewerteten bzw. vorausgewerteten Daten nur wenig Speicher benötigen.

Es ist auch möglich, sensornahe Elektronikteile durch Energie-Harvesting zu versorgen. Dies wird dadurch begünstigt, dass die Datenraten gering sind, die Anzahl der Sensoren niedrig ist und im Übrigen auch nur relativ selten Daten übertragen werden müssen. Es können also per se bekannte Mittel vorgesehen werden, um ein Energie-Harvesting vorzunehmen, etwa aus der Bewegung des Benutzers.

Weiter ist es möglich, eine Kalibrierung vorzunehmen, um aus den druckzonenweise erhaltenen Kennwerten auf eine tatsächlich gegebene Reallast zu schließen. Eine solche Kalibrierung kann statisch durchgeführt werden, weil eine Standbelastung ohne Weiteres an die beim Laufen auftretenden dynamischen Belastungen gekoppelt ist. Gegebenenfalls kann zusammen mit einer Kalibrierung auch ein zulässiger Sollwert bzw., bei Heilungsprozessen, ein Sollwertverlauf zulässiger Maximalbelastungen bzw. Kennwertschwellen vorgegeben werden. Einleuchtender Weise können solche Sollwertverläufe aber auch kalibrationsunabhängig eingestellt werden.

Es sei erwähnt, dass neben den belastungserfassenden Orthesen auch Einlegesohlen hierfür bzw. für z.B. postoperativ zu tragendes Schuhwerk als solche Schutz genießen sollen; dabei wird dann die Einlegesohle die Sensorelemente und zumindest einen Teil der die Sensorsignale auswertenden Elektronik und/Verbindungen für die Weiterleitung von Sensorsignalen bzw. konditionierten Sensorsignalen umfassen. Weiter kann ein Einlegesohlenteil, beispielsweise eine in eine Einlegesohle einzubauende Schicht mit den Sensorelementen und zumindest einem Teil der die Sensorsignale austretenden Elektronik und Querstrich oder Verbindung für die Weiterleitung von Sensorsignalen bzw. konditioniert Sensorsignalen für z.B. orthopädische Schumacher bereitgestellt werden. Auch hierfür wird Schutz beansprucht.

Die Erfindung wird im Folgenden nur beispielhaft anhand der Zeichnung beschrieben. In dieser ist dargestellt durch:
- Figur 1: eine belastungserfassende Orthese nach der vorliegenden Erfindung:
- Figur 2: einen horizontaler Schnitt durch eine Sensor-Einlegesohle;
- Figur 3: eine Auswerteelektronik für den Sensor von Figur 2;
- Figur 4: eine Einheit zur Erzeugung der Warnsignale;
- Figur 5a-c: eine Veranschaulichung der Kalibrierung eines Fußsohlensensors für eine belastungserfassende Orthese nach Figur 1.

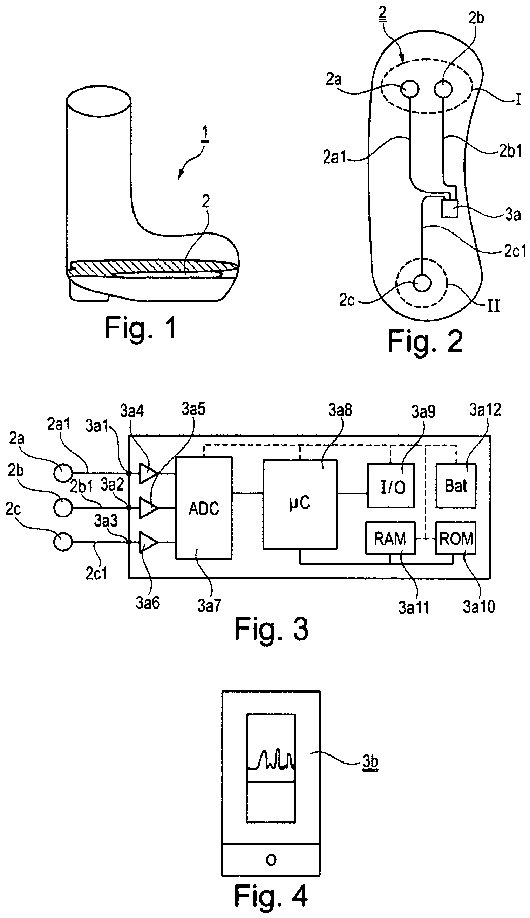

Nach Figur 1 umfasst eine allgemein mit 1 bezeichnete belastungserfassende Orthese l einen Sensorsignale erzeugenden Sensor 2, der Sensorelemente 2a, 2b, 2c (vgl. Figur 2) in Druckzonen, angedeutet durch gestrichelte Kreise I und II aufweist, und eine Sensorsignale auswertende Elektronik 3 dafür, die dazu ausgebildet ist, auf für den Orthesenbenützer kritische Belastungen hinzuweisen, wobei die Sensorsignale auswertende Elektronik 3a. 3b (vgl. Figur 4) dazu ausgebildet ist, Kennwerte zu mittels der Sensorelemente 2a, 2b, 2c druckzonenweise erfassten Druckbelastungsereignissen zu ermitteln, einen Summenwert, in welchen die Kennwerte nach Größe derart gewichtet eingehen, dass wenigstens 3 unterschiedliche Gewichtungen verwendet werden, zu bilden und ein Hinweissignal zu erzeugen, wenn der Summenwert eine bestimmte Größe überschreitet.

Die belastungserfassende Orthese I ist im dargestellten Ausführungsbcispiel eine hier z.B.nach Schienbeinbruch verwendbare Orthese, in der die Knochen nach dem Bruch in richtiger Position zueinander gehalten werden, damit sie korrekt wieder zusammenwachsen. Während des Heilungsprozesses ist es dem Benützer möglich, mit der Orthese 1 zu gehen, wobei er jedoch Sorge dafür zu tragen hat, dass er beim Gehen den verheilenden Bruch nicht überlastet. Deswegen wird er sich typisch mit Gehhilfen wie Krücken oder dgl. abstützen, um die Belastung auf dem heilenden Bein gering zu halten. Abhängig vom Fortschreiten der Heilung wird die maximal zulässige Belastung mit der Zeit wieder zunehmen, bis der Benützer sein Bein wieder voll belasten kann und die belastungserfassende Orthese nicht mehr benötigt. Bis zu diesem Zeitpunkt ist einerseits eine regelmäßige, jedoch geringe Belastung nach dem medizinischen Kenntnisstand sinnvoll zur Förderung des Heilungsprozesses; gleichzeitig sind Überlastungen zu vermeiden.

Derartige Überlastungen können auftreten, weil der Benutzer systematisch die Schwäche der Heilungsstelle ignoriert und etwa eine Gehhilfe wie einen Krückstock nur einseitig statt wie ärztlich empfohlen zweiseitig einsetzt, weil er schwere Dinge trägt oder dergleichen. Es kann aber auch sein, dass er etwa bei normalem vorsichtigem Gehen stolpert und sich mit dem schwachen Bein abfangen muss, was ebenfalls zu Druckbelastungen, die kritisch sind, führen kann.

Der Sensor 2 ist nun mit den in Druckzonen I und II angeordneten Sensorelementen 2a bis 2c in einer Einlegesohle für die belastungserfassende Orthese eingearbeitet. Die Einlegesohle kann eine Standardeinlegesohle sein, die mit einem trittdämpfenden Schaumstoff oder Polymerrnaterial aufgebaut ist, erforderlichenfalls wärmende Beschichtungen zum Fuß hin aufweist, und in per se bekannter Weise zur Verringerung von Fußschweiß und dgl. atmungsaktiv gebildet ist.

Es sei insbesondere darauf hingewiesen, dass die Sensorelemente 2a bis 2c und der in der Einlegesohle vorzusehende Teil 3a der auswertenden Elektronik sowie die Verbindungsleitungen dafür und/oder die Anschlüsse und/oder die Energieversorgungen nur wenig Raum einnehmen und insbesondere nur eine geringe Fläche der Einlegesohle überdecken bzw. einnehmen. Zudem ist es möglich, die einzelnen, geringfügig größerflächigen Komponenten, nämlich die Sensorelemente 2a, 2b, 2c sowie den Teil 3a der auswertenden Elektronik voneinander beabstandet anzuordnen und nur sehr dünne und zudem flexible Leitungen dazwischen anzuordnen. Dies verbessert die Atmungsaktivität einer entsprechenden Einlegesohle, weil die beeinträchtigte, durch die Komponenten nicht für Atmungsaktivität verfügbare Fläche gering bleibt. Die Verwendung von Flexfolien auch als Träger für in der Einlegesohle vorgesehene integrierte Schaltkreise sei als möglich und bevorzugt offenbart.

Zudem ist es möglich, die Sensorelemente unterhalb einer dem Fuß zugewandten Deck- und Dämpfschicht anzuordnen, insbesondere, wie in Figur 1 gezeigt, unmittelbar einem härteren, d.h. weniger elastischen Sohlenbereich der Orthese zugewandt. Dies ist schon deshalb vorteilhaft, weil damit eine unangenehme Druckbelastung für den Benützer aufgrund der im Vergleich zu der (Sohlen-) Umgebung etwas härteren Sensorelemente vermieden wird. Der Sensor führt also nicht zu Beeinträchtigungen hinsichtlich des Gehgefühls.

Überdies ist es möglich, eine untere Sohlenschicht zu verwenden, in welcher die entsprechenden Sensorelemente und der entsprechende Teil 3a der auswertenden Elektronik vorgesehen sind, und über dieser unteren bzw. untersten Einlegesohlenschicht eine an einen Fuß angepasste Einlegesohle individuell aufzubauen. Dass in einer solchen Situation letztlich die Sensorelemente usw. aber nicht unmittelbar an der Außenfläche zu liegen brauchen, sondern zu ihrem Schutz noch nach unten abgedeckt sein können, sei als vorteilhaft offenbart.

Ungeachtet der Möglichkeit, handwerklich individuell angepasste Einlegesohlen zu erzeugen, wird in besonders vorteilhaften Ausführungsformen eine standardisierte Einlegesohle verwendet, die lediglich nach linkem bzw. rechtem Fuß sowie einer Schuhgröße bzw. Schuhgrößengruppe unterschieden sein muss. Zusätzlich können, insbesondere dann, wenn insgesamt sehr große Stückzahlen gefertigt werden, Unterschiede gemacht werden, um empfindlichere oder weniger empfindliche Sensorelemente für leichtere oder schwerere Benutzer gleicher oder unterschiedlicher Schuhgrößen bereitzustellen; dass anstelle unterschiedlich empfindlicher Sensorelemente auch Druck unterschiedlich stark verteilende Deckschichten bzw. Unterschichten verwendet werden können, sei erwähnt.

Die Druckzonen I und II sind so gewählt, dass die bei Gehen und Stehen des Benützers an der Ferse bzw. dem Fußballen auftretende Belastung optimal erfasst werden kann. Es kann der Sensor 2c beispielsweise dort angeordnet werden, wo bei einer Vielzahl gesunder Benutzer im Mittel die höchste Belastung im Fersenbereich auftritt, wenn diese stehen oder gehen. Die größte Belastung im Stand für eine Vielzahl von Benützern kann beispielsweise bereits durch herkömmliche Blaupausentechnik ermittelt werden, wobei sich ein Benützer auf ein unterseitig blau eingefärbtes Stück Kopierpapier stellt, unter welchem ein weißes Stück Papier angeordnet ist. Die bei typischen Benützern stärksten Belastungen können dann fotogrammetrisch oder dergleichen ausgewertet und gemittelt werden. Einleuchtenderweise ist auch eine Auswertung mit moderneren Mitteln möglich, wenngleich nicht zwingend erforderlich. Der Teil 3a der auswertenden Elektronik, der an bzw. in oder auf der Einlegesohle angeordnet ist, ist nun wie folgt ausgebildet und wirksam:
Die von den Sensorelementen 2a, 2b, 2c über Leitungen 2a1, 2b1, 2c1 erhaltenen Signale werden von Schnittstellen 3a1, 3a2, 3a3 zu derzeit hier als Verstärker angedeuteten Signalkonditionierungsstufen 3a4, 3a5, 3a6 geführt, wo sie verstärkt und erforderlichenfalls gefiltert werden, beispielsweise, um Rauschanteile durch hochfrequente Komponenten zu reduzieren usw. Es kann auch eine Impedanzanpassung erfolgen, was insbesondere dort vorteilhaft ist, wo die Sensorelemente 2a bis 2c als Widerstandselemente gebildet sind, deren elektrischer Widerstand sich mit einwirkendem Druck ändert. Entsprechende Sensorelemente sind bekannt, es sei aber darauf hingewiesen, dass andere, druckempfindliche Elemente gleichfalls verwendbar sind, wie beispielsweise Dehnungs-Meßsensoren usw.

Die konditionierten Sensorsignale werden an einen ADC 3a7 gespeist, der wahlweise genügend Eingänge aufweist, die parallel analog-digital-gewandelt werden können; bevorzugt wird aber zwischen unterschiedlichen Eingängen zykliert oder durchgewechselt. Es sei darauf hingewiesen, dass die konditionierten Sensorsignale nicht zwingend einzeln in dedizierte Eingänge eines Analog-Digital-Konverters gespeist werden müssen, sondern daß es gleichfalls auch möglich wäre, dem ADC 3a7 einen Multiplexer vorzuschalten oder dergleichen. Es sei auch darauf hingewiesen, dass herkömmliche Analog-Digital-Konverter ohne Weiteres in der Lage sind, mit mehreren 10 kHz selbst dann abzutasten, wenn es sich um preiswerte Analog-Digital-Konverter handelt, was selbst dann, wenn zwischen verschiedenen Sensoren durchzykliert werden soll, angesichts der eher langsamen Bewegungen gerade für körperlich beeinträchtigte Benutzer im Regelfall mehr als ausreichend ist. Dies gilt selbst dort, wo kurze Belastungsspitzen, etwa durch heftiges plötzliches Aufstampfen oder die Notwendigkeit, sich stolpernd abzufangen, auftreten. ADC-Genauigkeiten von 8 bit, bevorzugt 10 oder 12 bit sind ohne weiteres ausreichend.

Die Digitalsignale aus dem Analog-Digital-Wandler 3a7 werden an einen Mikrokontroller 3a8 gespeist, dessen Ausgang wiederum an eine I/O-Schnittstelle 3a9 geführt ist. Dem Mikrokontroller 3a8 ist ein nichtflüchtiger Lesespeicher ROM 3a10 zugeordnet sowie ein Speicher für wahlweisen Zugriff RAM 3a 11. Die entsprechenden Teile werden von einer Leistungsversorgung 3a12 versorgt, wie durch die von der Leistungsversorgung 3a12 zu den einzelnen Einheiten 3a1 bis 3a11 führenden gestrichelten Leitungen angedeutet.

Die Batterie braucht nicht unmittelbar auf einer Platine bzw. einer Flexfolie dicht bei den anderen Komponenten Energie zu speichern. Abhängig davon, wie lange die Batterie die Schaltkreise mit Leistung versorgen soll, kann es vorteilhaft sein, eine etwas größere Energiespeicheranordnung, beispielsweise eine Knopfzelle, etwas entfernt anzuordnen, insbesondere in leicht wechselbarer Weise, und/oder es kann vorgesehen werden, eine Energie-Harvesting-Anordnung vorzusehen, die Energie aus den Schrittbewegungen gewinnt, und zwar in zur Versorgung der Anordnung ausreichender Weise.

Es sei erwähnt, dass der Mikrokontroller 3a8 übliche Schaltkreise wie beispielsweise einen Zeitgeber zur Erfassung einer aktuellen Zeit aufweist, so dass Druckereignis- bezogene Daten bzw. Kennwerte mit Zeitmaßen versehen abgelegt werden können.

Im ROM 3a10 sind Programmmodule abgelegt, die es bei Abarbeitung auf dem Mikrokontroller 3a8 erlauben, in den Verläufen der aus dem ADC erhaltenen Signale Schritte zu identifizieren, und schrittweise oder zeitperiodenweise Druckbelastungsereignisse zumindest druckzonenweise auf den Sensorelementen 2a, 2b für die Druckzone 1 bzw. 2c für die Druckzone II zu erkennen. Weiter können im ROM 3a10 Informationen abgelegt sein, um den Mikrokontroller 3a8 dazu zu veranlassen, die digitalisierten Kennwerte je Schritt oder je Zeitperiode, insbesondere bei Stehen je Zeitperiode, auf Spitzenwerte zu untersuchen und diese festzustellen.

Der ADC 3a7 braucht je Sensor nur etwa eine Abtastfrequenz von beispielsweise 100 Hz, was ohne Weiteres ausreicht, gerade bei körperlich beeinträchtigten Personen, ohne Probleme 15-30 Werte je Schritt zu erfassen. Dies erlaubt es, bei der Spitzenwertbestimmung gegebenenfalls auch mehrere Werte gemittelt zu betrachten, beispielsweise um eine ungefähre Spitze herum, um so Rauscheffekte, samplingbedingte Effekte usw. zu verringern. Es können beispielsweise 3-6 Werte zusammengefasst werden und es kann daran jeweils die Spitzenbelastung erfasst werden. Der Mikrokontroller 3a8 ist weiter dazu programmierbar, entweder vor der Spitzenwertberechnung und/oder nach der Spitzenwertberechnung, einen Mittelwert der beiden Sensorelemente 2a, 2b, die zur Druckzone 1 gehören, zu bestimmen. Dies kann entweder durch eine gemeinsame Auswertung der Sensorelement-Signale erfolgen, durch die also, anders als gezeigt, gemeinsame Signalkonditionierung und Signalwandlung, oder aber es wird für jedes Sensorelement 2a, 2b jeweils separat eine Druckspitze festgestellt, um dann die Druckspitzen miteinander zu verrechnen, zum Beispiel durch Mittelwertbildung. Letzteres hat den Vorteil, dass kritische oder atypische Belastungen noch besser erkannt werden. Dass auch anstelle einer Druckspitzen-Mittelung die zeitlichen Mittelwerte der in jedem Sensorelement erfassten Druckbelastungen über die Sensorelemente einer Druckzone verrechnet, insbesondere gemittelt werden können, sei erwähnt.

Weiter kann der Mikrokontroller 3a8 dazu ausgebildet bzw. programmiert sein, die ADC-Werte, die als Ausgangssignale aus dem Analog-Digital-Konverter erhalten werden, zu korrigieren. Es wird nämlich einsichtig sein, dass es wünschenswert ist, preiswerte Sensoren anbieten zu können, dies kann aber zur Folge haben, dass die Reproduzierbarkeit von Daten abnimmt. Gerade deshalb, weil Überlastungen sicher vermieden werden sollen, ist es dann vorteilhaft, eine tatsächlich auftretende Druckbelastung exakter zu erfassen.

So kann etwa je nach exakter Fußhaltung, Ballenfläche usw. eine unterschiedliche Belastung trotz gleicher Auftrittskraft auf den Boden an jedem der Sensorelemente 2a, 2b auftreten. Dies kann kompensiert werden, indem die belastungserfassende Orthese nach Einlegen der Einlegesohle vom Benützer mit einer definierten Kraft belastet wird. Eine solche Kraft kann durch Auftreten auf eine Personenwaage oder dgl. gut eingeschätzt werden. Es zeigt sich dabei, dass wiederholtes Auftreten mit gleicher Kraft zwar bei ein und demselben Benutzer zu reproduzierbar gleichen Größen der Druckbelastungsereignissen führt, dass aber von Benützer zu Benützer Unterschiede beobachtet werden, und zwar zum Teil hinsichtlich der Gesamtgröße der jeweiligen Ereignisse, und zum Teil hinsichtlich der Verteilung der Druckbelastung auf die verschiedenen Sensorelemente. Es ist einsichtig, dass dies unter anderem mit Stand- bzw. Gehgewohnheiten sowie z.B. der Form des Fußes zu tun hat, beispielsweise aufgrund hornhautbedingter Verhärtungen im Ballenbereich usw.. Es ist demnach ausreichend, mit statischen Kräften bzw. Belastungen zu kalibrieren, obwohl eine Überlastung beim Gehen durch die dabei auftretenden dynamischen Kräfte zu erwarten ist.

Es ist also vorteilhaft, zunächst Messwerte aufzunehmen und anhand dieser die Druckereignissignale aus den Sensorelementen zu kalibrieren. Dies erlaubt es, eine präzisere Gewichtung der Kennwerte vorzunehmen, insbesondere für besonders leichte Personen und/oder für Personen mit atypischen Fußformen.

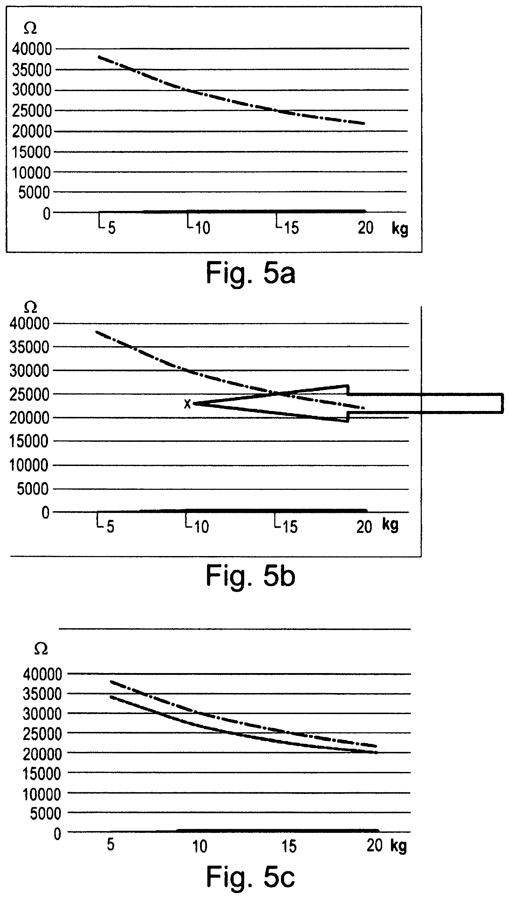

Die Anordnung ist daher dazu ausgebildet, über ein Bediengerät wie ein Handy, vgl. Figur 4, und die I/O-Schnittstelle 3a9 in einen Kalibriermodus geschaltet zu werden, in welchem die Drucksensorsignale gespeichert werden, und dann einen tatsächlich erreichten, zugehörigen Belastungswert über die Schnittstelle 3a9 zu empfangen. Es ist dafür möglich, einzelne Werte für einzelne Druckbelastungen zu bestimmen und anhand dieser Einzelwerte die gesamte Kurve zu kalibrieren, wie in Figur 5 angedeutet. Dabei ist in Figur 5a eine Standard-Kalibrierkurve dargestellt, die den Widerstandsverlauf abhängig von einer Belastung des Sensors darstellt; in Figur 5b ist gezeigt, dass ein Messwert für einen bestimmten Benützer bei einer gemessenen Belastung von dort 10 kg deutlich geringere Widerstandswerte zeigt als nach Normkurve zu erwarten, und in Figur 5c ist eine dementsprechend korrigierte Kalibrierkurve dargestellt, die aus einer entsprechenden Kalibriertabelle bestimmt wurde.

Eine solche Kalibrierung kann vorgenommen werden wie folgt: Zunächst wird eine Belastung bestimmt, die für den Benutzer unkritisch ist, mit der also ein heilendes Bein beliebig lange belastet werden darf. Dann tritt der Benutzer mit nur dem Bein, an dem sich die Orthese befindet, auf eine hinreichend empfindliche Waage, wobei er das entsprechende Bein immer stärker belastet, bis die vorgegebene Belastung erreicht ist. (Die auf der Waage angezeigte Belastung wird sich einleuchtender Weise zusammensetzen aus einerseits der Belastung durch die Orthese und andererseits durch das Aufstehen des Beines in der Orthese; gegebenenfalls kann dementsprechend eine um das Gewicht der Orthese höhere Belastung auf der Waage gewählt werden oder es wird eine Vorab-Messung des Orthesengewichtes vorgenommen.). Sobald die gewünschte Belastung auf der Waage erreicht ist, kann eine Kalibrierung ausgelöst werden. Dies kann durch den Benutzer selbst geschehen, indem dieser ein Smartphone oder dergl. mit einer geeigneten App bedient, oder durch eine Hilfsperson. Es sei erwähnt, dass gegebenenfalls auch eine Waage ein entsprechendes Signal auslösen könnte.

Aus der Kalibration an einem festen Gewichtswert kann dann von einem Sensorwert auf die tatsächliche Belastung geschlossen werden. Mit einer bekannten allgemeinen Kalibrationskurve, in welche einerseits die tatsächlichen Sensorsignalwerte bei einer bekannten Belastung eingehen, und mindestens einem benutzerspezifischen Kalbrierwert kann eine benutzerspezifische Kalibriertabelle (bzw., wie dargestellt, Kalbrierkurve) bestimmt werden.

Es sei erwähnt, dass im Regelfall die Aufnahme eines einzelnen benutzerspezifischen Kalibrierwertes ausreicht, weil die Belastungsverteilung sich im für die Heilung kritischen Lastbereich nicht signifikant mit zunehmenden Lasten ändert.

Die Kalibrierung respektive eine Kalibriertabelle kann im RAM 3al 1 abgelegt werden.

Es ist also möglich, die elektronischen Messsignale mit allenfalls sehr geringem Aufwand auf tatsächlich bei einem bestimmten Benutzer auftretende Belastungen zu beziehen. Dabei ist nützlich, dass im Regelfall schon mit wenigen Sensorelementen das bei ein und demselben Benutzer am Fuß bestimmte Belastungsmuster auch bei unterschiedlichen Belastungen gleich bleibt und mit der Gesamtbelastung gut skaliert.

Weiter ist die Anordnung dazu ausgebildet, über die Schnittstelle 3a9 einen Maximalwert für die Belastung zu erhalten und im RAM 3a11 abzulegen. Es sei erwähnt, dass es bevorzugt und möglich ist, anstelle eines zeitlich festen Wertes, der als absolute Obergrenze einer Belastung definiert ist, einen zeitlichen Obergrenzenverlauf festzulegen, der angibt, wie die maximal zulässige Belastbarkeit über Tage und/oder Wochen hinweg zunehmen soll. Weiter sei erwähnt, dass entweder aus der festgelegten Obergrenze einer Belastung die geringeren Schwellwerte abgeleitet werden können, etwa durch prozentuale Reduktion um 10%.20%.30% oder 25% und 50% bzw. um z.B. 1/5, 1/4, 1/3 und 1/2, oder aber, dass geeignete zusätzliche Schwellwerte mitablegbar sind, was den Vorteil bietet, dass eine bessere Anpassung an bestimmte Patienten möglich ist. Dadurch ist insbesondere eine präzisere Anpassung der weiteren Schwellwerte an die Maximalbelastung abhängig vom jeweiligen Eingriff, also dem spezifischen Bruch, spezifischen Operation usw., ermöglicht.

Der Mikrokontroller 3a8 ist weiter dazu ausgebildet, im RAM 3a11 einerseits die erfassten Spitzenwerte der Druckbelastung abzulegen, gegebenenfalls zeitlich gemittelt über die Druckspitze und räumlich über die Druckzone, und zwar bevorzugt zusammen mit Zeitinformation; außerdem werden einzelne Werte, insbesondere episodenweise, sowie besonders große einzelne Spitzenereignisse abgelegt. Die Ereignisse werden zugleich bereits in der Sohle dahingehend bewertet, ob sie einzeln oder zusammengenommen auf eine kritische Be- bzw. Überlastung hindeuten. Dies kann dort, wo eine vollständige erfindungsgemäße Auswertung nicht vorgenommen werden soll, etwa um den sohlennahen Rechenaufwand geringer zu halten. z.B. dergestalt geschehen, dass lediglich eine konservative Abschätzung vorgenommen wird, etwa mit gleichhohen Gewichtungen für alle eine der Schwellen überschreitenden Belastung oder eine Summenwertbildung ohne Berücksichtigung eines Abklingverhaltens vorgenommen wird.

Die Anordnung ist dazu ausgebildet, die Daten per Funk, insbesondere per Bluetooth, an ein Smartphone, eine Smartwatch oder dgl. Mobilgerät zu übertragen. Dazu werden in üblicher Weise Verbindungen aufgebaut usw. Dies geschieht entweder auf Aufforderung vom Mobilgerät her oder aktiv von der Einlegesohle, insbesondere, wenn dort der Ereignisspeicher bereits weitgehend gefüllt ist oder wenn besonders kritische oder viele nahezu kritische Ereignisse beobachtet werden.

Es sei im Übrigen darauf hingewiesen, dass das Mobilgerät einerseits dem Patienten der belastungserfassenden Orthese ein Hinweis-Signal ausgeben kann; darauf, dass eine solche Signalausgabe nicht unmittelbar durch das Mobilgerät erfolgen braucht, sei hingewiesen. So ist es beispielsweise möglich, dass die belastungserfassende Orthese selbst nur mit dem Smartphone des Benutzers kommuniziert (wobei in bevorzugten Varianten insbesondere eine Kommunikation auch zu Geräten aufgebaut werden kann, die von Ärzten, Physiotherapeuten, orthopädischen Schuhmachern usw. zum anfänglichen oder wiederholten Einstellen von Grenzwerten und Gewichtungen benützt werden), dass aber das Smartphone selbst wiederum mit anderen Geräten verbunden sein kann, beispielsweise einem Hinweissignal-Geber, etwa einer Smartwatch mit Vibrationsalarm, die dem Benutzer am Handgelenk ein taktiles Hinweissignal gibt. Weiter ist alternativ und oder zusätzlich möglich, dass Signale vom Mobilgerät wie dem Smartphone an eine Zentrale weitergeleitet werden. Dies muss nicht zwingend in Echtzeit geschehen, insbesondere dort nicht, wo lediglich eine statistische Auswertung erfolgen soll, etwa um zusätzliche Erkenntnisse über allgemeine Heilungsverläufe von Patientenkohorten zu gewinnen oder um in größeren Abständen wie Tage-oder wochenweise zu überprüfen, ob ein Patient sich hinreichend bewegt, sich überlastet hat und so weiter. Dabei kann dann auch eine zentrale Konfigurierung bzw. Re-Konfigurierung aus der Ferne bewirkt werden, sodass der Patient selbst von derartigen Konfigurationsaufgaben entlastet ist, aber gleichwohl eine Anpassung seiner Hilfsmittel an eine seit einem Eingriff oder Unfall verstrichene Zeitdauer bzw. beobachtete diagnostische Erfolge vorgenommen wird. Die bei großen Zahlen von Patienten anfallenden Daten helfen dann auch, typisch noch zulässige Belastungen. Grenzwerte und so weiter festzulegen. Wo derzeit noch mehr oder minder vorsichtig akzeptable Grenzwerte durch einen behandelnden Arzt oder Physiotherapeuten geschätzt werden müssen, kann unter Rückgriff auf eine Datenbank ein Erfahrungen mit anderen Patienten besser berücksichtigender Wert vorgeschlagen oder vorgegeben werden, beispielsweise ein Wert, der bei Patienten ähnlichen Alters oder ähnlicher physischer Konstitution und vergleichbaren Verletzungen unkritisch war und nicht zu Problemen geführt hat, auch nicht unter Berücksichtigung gelegentlich auftretender Überschreitungen der Grenzwerte. Es sei darauf hingewiesen, dass eine Fern-Konfigurierung sowohl durch medizinisch geschultes Personal wie Ärzte oder Physiotherapeuten vorgenommen werden kann, als auch gegebenenfalls maschinell, sofern dem keine zulassungstechnischen Hindernisse entgegenstehen. Dass auch bei Fern-Konfigurierung Besonderheiten berücksichtigt werden können, etwa eine Anpassung der Grenzbelastung bei sehr schweren Patienten, wird ohne weiteres verständlich sein.

Es sei im Übrigen weiter darauf hingewiesen, dass basierend auf der Analyse der Belastungsdaten nicht nur Warnsignale erzeugt werden können, sondern dass gegebenenfalls auch die Möglichkeit besteht, zu erkennen, inwieweit ein Patient weiteres Training benötigt. So lassen sich etwa Ereignisse gut erkennen, bei denen der Patient eine Treppe hinauf- oder hinab geht. Es ist bekannt, dass beim Treppensteigen besonders häufig Probleme auftreten können; daher wird das Treppensteigen in vielen Fällen separat trainiert. Wenn, was unter Verwendung der belastungserfassenden Orthese möglich ist, festgestellt wird, dass gerade bei der Treppenbenutzung Probleme auftreten, kann der Patient etwa aufgefordert werden, Treppensteigen gezielt zu trainieren. Prinzipiell ist dies beispielsweise möglich, indem Belastungsdaten an eine Zentrale übermittelt werden und dort - bevorzugt automatisch - analysiert werden. Das Ergebnis einer automatischen Analyse kann dann einem erfahrenen Berufsträger, etwa einem Physiotherapeuten, gezeigt werden, der dann erforderlichenfalls mit dem Patienten Kontakt aufnimmt und ein (Nach-) Training vereinbart. Gegebenenfalls kann dies auch vollautomatisch geschehen. Eine Alternative zur Datenübertragung an eine Zentrale und die Analyse der Daten in der Zentrale besteht darin, lokal, etwa auf dem Smartphone des Benutzers, eine Analyse durchzuführen und es kann dann, sofern die Analyse zeigt, dass besondere Problembereiche bei einem Patienten vorhanden sind, etwa ungenügendes Treppensteigen, erforderlichenfalls dem Patienten die Anregung oder Möglichkeit gegeben werden, einen Physiotherapeuten für ein Nach-Training zu kontaktieren, sich Trainingsvideos anzusehen oder dergleichen. Eine solche lokale Auswertung ist einsichtiger Weise besonders dort vorteilhaft, wo Patienten besonders um den Schutz ihrer Daten besorgt sind.

Es sei weiter darauf hingewiesen, dass mit der belastungserfassenden Orthese gemäß der vorliegenden Erfindung bereits Daten gesammelt wurden, die zeigen, dass selbst bei jenen Patienten, bei welchen vom Arzt als kritisch eingestufte Belastungen zwar nicht häufiger als bei anderen Patienten überschritten wurden, aber gleichwohl regelmäßig sehr hohe Belastungen unterhalb der vom Arzt als maximal zulässig befundenen Belastung aufgetreten sind, Probleme beobachtet wurden. Dies zeigt einerseits, dass eine häufige, hohe, wenn auch für sich noch nicht kritische Belastungen erfassende Orthese der vorliegenden Erfindung dazu beitragen kann, Nach-Behandlungen signifikant zu reduzieren und zugleich Grenzbelastungen besser vorgeben zu können.

Die Auswerteeinheit im Mobilfunkgerät ist nun dazu ausgebildet, auf den Empfang von Daten einen Summenwert zu bilden, in welchen die Kennwerte aus der Einheit 3a nach Größe gewichtet derart eingehen, dass wenigstens 3 unterschiedliche Gewichtungen verwendet werden, und ein Warnsignal zu erzeugen, wenn der Summenwert zu groß ist. Die auswertende Elektronik 3b im Mobilfunkgerät (Figur 4) ist dazu ausgebildet, die erhaltenen Kennwerte mit Schwellwerten zu vergleichen und gegebenenfalls zu klassifizieren. Dies kann insbesondere von Schritt zu Schritt geschehen.

Es ist möglich, eine Übertragung von Daten stets nur dann auszulösen, wenn eine erste Schwelle ausweislich einer (Vor)-Auswertung im sohlennahen Bauteil von 3a überschritten wird und/oder insgesamt eine (Vor)-Auswertung auf dem fußsohlennahen Teil 3a zeigt, dass kritische Belastungen wiederholt überschritten werden.

Auch kann eine Gesamtauswertung sohlennah erfolgen, und nur dann, wenn ein etwa akustisches oder Vibrations-Signal dem Benützer angezeigt werden soll, eine Übertragung von geeigneten Daten an die Einheit 3b erfolgen. Dies spart aufgrund des geringeren Datenübertragungsaufwandes regelmäßig insgesamt Energie. Dass zur Vereinfachung die Gesamtelektronik unmittelbar an der Orthese angeordnet sein kann, sei aber erwähnt, In einem solchen Fall kann an der Orthese ein akustisch, vibratorisch und/oder optisch wirkender Alarm erzeugt werden und/oder im Alarmfall eine bevorzugt drahtlose Übertragung an einen Alarmgeber ausgelöst werden, wie an ein Smartphone oder an eine Smartwatch.

Mit der Erfindung ist es möglich, energiesparend und mit geringem Aufwand ein hohes Maß an Sicherheit bei gleichzeitig großem Komfort für den Benützer zu gewährleisten.

Vorstehend beschrieben wurde somit unter anderem, jedoch nicht ausschließlich, eine belastungserfassende Orthese mit einem Sensorsignale erzeugenden Standardsensor, der nicht mehr als vier Druckzonen und je Druckzone nicht mehr als vier Sensorelemente aufweist, einer Sensorsignale auswertenden Elektronik dafür, und einer Individualanpassung an individuelle Patienten, wobei die Sensorsignale auswertende Elektronik dazu ausgebildet ist, auf für das Individuum kritische Belastungen hinzuweisen, wobei die Sensorsignale auswertende Elektronik dazu ausgebildet ist, die Druckbelastung der Sensorelemente zumindest druckzonenweise abzutasten und zu gewichten, eine Schrittidentifikation zu durchlaufen. Lastspitzen für jeden Schritt durch Vergleich mit einer Mehrzahl Schwellwerte zu klassifizieren und einen Summenwert, in welchen Lastspitzen derart gewichtet eingehen, dass höhere Lastspitzen höhere Gewichtungen erhalten, zu bilden und ein Hinweis- bzw. Warnsignal zu erzeugen, wenn der Summenwert eine bestimmte Größe überschreitet, d.h, zu groß wird.

## Patentansprüche

1. Belastungserfassende Orthese (1) mit
einem Sensorsignale erzeugenden Sensor (2), der Sensorelemente (2a,2b,2c) in Druckzonen (I,II) aufweist,
und
einer Sensorsignale auswertenden Elektronik (3) dafür, die dazu ausgebildet ist, auf kritische Belastungen hinzuweisen,
**dadurch gekennzeichnet, dass**
die Sensorsignale auswertende Elektronik (3) dazu ausgebildet ist, Kennwerte zu mittels der Sensorelemente (2a,2b,2c) zumindest druckzonenweise erfassten Druckbelastungsereignissen zu ermitteln, einen Summenwert, in welchen die Kennwerte nach Größe derart gewichtet eingehen, dass wenigstens drei unterschiedliche Gewichte verwendet werden, zu bilden und ein Hinweissignal zu erzeugen, wenn der Summenwert eine festgelegte Größe überschreitet.

2. Belastungserfassende Orthese (1) nach dem vorhergehenden Anspruch, **dadurch gekennzeichnet, dass** die Sensorsignale auswertende Elektronik (3) weiter dazu ausgebildet ist, dass Kennwerte durch Vergleich mit einer Mehrzahl Schwellwerte klassifiziert werden, bevorzugt je Schritt, und die Klassifikation derart zur Gewichtung herangezogen wird, dass zu größeren Kennwerten hin stärkere Gewichtungen erhalten werden.

3. Belastungserfiissende Orthese (1) nach einem der vorhergehenden Ansprüche,
**dadurch gekennzeichnet, dass** die Sensorsignale auswertende Elektronik (3) dazu ausgebildet ist, eine zeitliche Dichte von Kennwerten oberhalb bestimmter Schwellwerte bei der Summenwertbildung zu berücksichtigen,
bevorzugt durch Abklingenlassen von Beiträgen von Druckbelastungsereignissen zu einem Summenwert, insbesondere bevorzugt durch schnelleres oder alleiniges Abklingenlassen der Beiträge nur kleinerer oder kürzerer Druckbelastungsereignisse.

4. Belastungserfassende Orthese (1) nach einem der vorhergehenden Ansprüche, **dadurch gekennzeichnet, dass** der Sensorsignale erzeugende Sensor (2) ein Standardsensor bzw. ein für eine bestimmte Schuhgröße bzw. Schuhgrößengruppe standardisierter Sensor ist, und bevorzugt Teil einer Einlegesohle ist.

5. Belastungserfassende Orthese (1) nach einem der vorhergehenden Ansprüche, **dadurch gekennzeichnet, dass** nicht mehr als vier, bevorzugt lediglich nicht mehr als drei, insbesondere zwei Druckzonen (I,II) vorhanden sind.

6. Belastungserfassende Orthese (1) nach einem der vorhergehenden Ansprüche, **dadurch gekennzeichnet, dass** je Druckzone (I,II) nicht mehr als vier, insbesondere nicht mehr als zwei, insbesondere bevorzugt ein bzw. zwei Sensorelemente (2a,2b,2c) vorhanden sind.

7. Belastungserfassende Orthese (1) nach einem der vorhergehenden Ansprüche, **dadurch gekennzeichnet, dass** der Drucksensor in einer Belastungen vergleichmäßigenden und/oder in einer Druckspitzen dämpfenden Einlegesohle angeordnet ist, insbesondere einer an den Patienten angepassten Einlegesohle.

8. Belastungserfassende Orthese (1) nach einem der vorhergehenden Ansprüche, **dadurch gekennzeichnet, dass** die Elektronik (3) zumindest erste, sensornahe Schaltkreise und entferntere Schaltkreise aufweist, die über eine Drahtlosschnittstelle verbunden sind, insbesondere über Bluetooth und/oder WIFI und/oder **dadurch gekennzeichnet, dass** sie dazu ausgebildet ist, dass zumindest sensornahe Elektronik-Teile durch Energie-Harvesting versorgt werden.

9. Belastungserfassende Orthese (1) nach einem der vorhergehenden Ansprüche, **dadurch gekennzeichnet, dass** die Sensorsignale auswertende Elektronik (3) weiter dazu ausgebildet ist, die Kennwerte durch A/D-Wandlung von Druckbelastungs-Sensorelementsignalen zu erhalten und zumindest druckzonenweise zu gewichten, bevorzugt druckzonenweise gewichtete Werte vor Bestimmung der Kennwerte zusammenzufassen,
wobei
vorzugsweise eine Gewichtung auf eine erfasste Reallast, insbesondere auf eine statische Reallast bezogen ist.

10. Einlegesohle für eine belastungserfassende Orthese (1) mit einem Sensorsignale erzeugenden Sensor (2) , der Sensorelemente in Druckzonen (I,II) aufweist, und einer Sensorsignale auswertenden Elektronik (3) dafür, die dazu ausgebildet ist, auf kritische Belastungen hinzuweisen, **dadurch gekennzeichnet, dass** die Sensorsignale auswertende Elektronik (3) dazu ausgebildet ist, Kennwerte zu mittels der Sensorelemente (2a,2b,2c) zumindest druckzonenweise erfassten Druckbelastungsereignissen zu ermitteln, einen Summenwert, in welchen die Kennwerte nach Größe derart gewichtet eingehen, dass wenigstens drei unterschiedliche Gewichtungen verwendet werden, zu bilden und ein Hinweissignal zu erzeugen, wenn der Summenwert eine festgelegte Größe überschreitet.

## Claims

1. A loading-detecting orthosis (1) having
a sensor (2) generating sensor signals that has sensor elements (2a,2b,2c) in pressure areas (I,II),
and
a sensor signal-evaluating electronics (3) therefor designed to indicate critical loading,
**characterized in that**
the sensor signal-evaluating electronics is designed to ascertain characteristic values regarding pressure loading events detected at least per pressure area by way of the sensor elements (2a,2b,2c) , to form a sum value into which the characteristic values are incorporated by amount in weighted form such that at least three different weights are used, and to generate an alert signal when the sum value exceeds a stipulated amount.

2. The loading-detecting orthosis (1) as claimed in the preceding claim, **characterized in that** the sensor signal-evaluating electronics (3) is furthermore designed such that characteristic values are classified through comparison with a multiplicity of threshold values, preferably per step, and the classification is applied to the weighting such that greater weightings are obtained as the characteristic values become larger.

3. The loading-detecting orthosis (1) as claimed in either of the preceding claims, **characterized in that** the sensor signal-evaluating electronics (3) is designed to take into consideration a temporal density of characteristic values above particular threshold values in the sum value formation,
preferably by allowing contributions from pressure loading events to a sum value to subside, particularly preferably by allowing the contributions of only relatively small or relatively short pressure loading events to subside faster or solely.

4. The loading-detecting orthosis (1) as claimed in one of the preceding claims, **characterized in that** the sensor (2) that generates sensor signals is a standard sensor or a sensor standardized for a particular shoe size or shoe size group respectively, and is preferably part of an insole.

5. The loading-detecting orthosis (1) as claimed in one of the preceding claims, **characterized in that** no more than four, preferably only no more than three, in particular two pressure areas (I,II) are present.

6. The loading-detecting orthosis (1) as claimed in one of the preceding claims, **characterized in that** no more than four, in particular no more than two, particularly preferably one or two sensor elements (2a,2b,2c) respectively are present per pressure area (I,II).

7. The loading-detecting orthosis (1) as claimed in one of the preceding claims, **characterized in that** the pressure sensor is arranged in an insole that equalizes loading and/or damps pressure peaks, in particular an insole adapted to the patient.

8. The loading-detecting orthosis (1) as claimed in one of the preceding claims, **characterized in that** the electronics (3) has at least first circuits close to the sensors and more remote circuits that are connected via a wireless interface, in particular via Bluetooth and/or Wi-Fi and/or **characterized in that** it is designed such that at least electronic parts close to the sensors are supplied with power through energy harvesting.

9. The loading-detecting orthosis (1) as claimed in one of the preceding claims, **characterized in that** the sensor signal-evaluating electronics (3) is furthermore designed to obtain the characteristic values through A/D conversion of pressure loading sensor element signals and to weight them at least by pressure area, preferably to combine values weighted by pressure area before determining the characteristic values,
wherein
a weighting preferably relates to a detected real load, in particular to a static real load.

10. An insole for a loading-detecting orthosis (1) having a sensor (2) that generates sensor signals and that has sensor elements in pressure areas (I,II), and sensor signal-evaluating electronics (3) therefor that is designed to indicate critical loading, **characterized in that** the sensor signal-evaluating electronics (3) is designed to ascertain characteristic values regarding pressure loading events detected at least per pressure area by way of the sensor elements (2a,2b,2c) , to form a sum value into which the characteristic values are incorporated by amount in weighted form such that at least three different weightings are used, and to generate an alert signal when the sum value exceeds a stipulated amount.

## Revendications

1. Orthèse à détection de charge (1) comprenant
un capteur (2) qui génère des signaux de capteur et qui comporte des éléments capteurs (2a, 2b, 2c) dans des zones de pression (I, II),
et
une électronique d'évaluation de signaux de capteur (3) qui est conçue pour signaler des charges critiques,
**caractérisée en ce que**
l'électronique d'évaluation de signaux de capteur (3) est conçue pour déterminer des valeurs caractéristiques relatives à des événements de charge de pression détectés au moins dans des zones de pression au moyen des éléments capteurs (2a, 2b, 2c), former une valeur somme dans laquelle les valeurs caractéristiques sont prises en compte de manière pondérée en fonction de la dimension à l'aide d'au moins trois poids différents et générer un signal de signalement lorsque la valeur somme dépasse une dimension spécifiée.

2. Orthèse à détection de charge (1) selon la revendication précédente, **caractérisée en ce que** l'électronique d'évaluation de signaux de capteur (3) est en outre conçue pour classer des valeurs caractéristiques en les comparant à une pluralité de valeurs de seuil, de préférence pour chaque étape, et pour utiliser la classification dans la pondération de manière à obtenir des pondérations d'autant plus fortes que les valeurs caractéristiques sont plus grandes.

3. Orthèse à détection de charge (1) selon l'une des revendications précédentes, **caractérisée en ce que** l'électronique d'évaluation de signaux de capteur (3) est conçue pour prendre en compte une densité temporelle de valeurs caractéristiques au-dessus de valeurs de seuil déterminées lors de la formation de la valeur somme, de préférence par décroissance de la contribution d'événements de charge de pression à une valeur somme, de manière particulièrement préférée par décroissance plus rapide ou unique de la contribution seulement d'événements de charge de pression plus petits ou plus courts.

4. Orthèse à détection de charge (1) selon l'une des revendications précédentes, **caractérisée en ce que** le capteur (2), qui génère des signaux de capteur, est un capteur standard ou un capteur normalisé pour une dimension de chaussure déterminée ou un groupe de dimension de chaussure déterminé et fait de préférence partie d'une semelle intérieure.

5. Orthèse de détection de charge (1) selon l'une des revendications précédentes, **caractérisée en ce que** le nombre zones de pression (I, II) n'est pas supérieur à quatre, de préférence n'est pas supérieur à trois seulement, en particulier est de deux.

6. Orthèse de détection de charge (1) selon l'une des revendications précédentes, **caractérisée en ce que** le nombre d'éléments capteurs (2a, 2b, 2c) par zone de pression (I, II) n'est pas supérieur à quatre, en particulier n'est pas supérieur à deux, de manière particulièrement préférée est de un ou deux.

7. Orthèse à détection de charge (1) selon l'une des revendications précédentes, **caractérisée en ce que** le capteur de pression est disposé dans une semelle intérieure qui uniformise les charges et/ou les pics de pression, notamment une semelle intérieure adaptée au patient.

8. Orthèse de détection de charge (1) selon l'une des revendications précédentes, **caractérisée en ce que** l'électronique (3) comporte au moins des premiers circuits proches du capteur et des circuits distants, qui sont reliés par le biais d'une interface sans fil, notamment par Bluetooth et/ou WIFI et/ou **caractérisée en ce qu'**elle est conçue pour qu'au moins des parties de l'électronique proches du capteur soient alimentées par récupération d'énergie.

9. Orthèse de détection de charge (1) selon l'une des revendications précédentes, **caractérisée en ce que** l'électronique d'évaluation de signaux de capteur (3) est en outre conçue pour obtenir les valeurs caractéristiques en effectuant une conversion A/N de signaux d'élément capteur de charge de pression et en les pondérant au moins par zones de pression, de préférence en rassemblant des valeurs pondérées par zones de pression avant de déterminer les valeurs caractéristiques,
une pondération étant de préférence liée à une charge réelle détectée, notamment à une charge réelle statique.

10. Semelle intérieure destinée à une orthèse à détection de charge (1) comprenant un capteur (2) qui génère des signaux de capteur et qui comporte des éléments de capteur dans des zones de pression (I, II), et une électronique d'évaluation de signaux de capteur (3) qui est conçue pour signaler des charges critiques, **caractérisée en ce que** l'électronique d'évaluation de signaux de capteur (3) est conçue pour déterminer des valeurs caractéristiques relatives à des événements de charge de pression détectés au moins dans des zones de pression au moyen des éléments de capteur (2a, 2b, 2c), former une valeur somme dans laquelle les valeurs caractéristiques sont prises en compte de manière pondérée en fonction de la dimension à l'aide d'au moins trois poids différents et générer un signal de signalement lorsque la valeur somme dépasse une grandeur spécifiée.
